# EUROPEAN PATENT APPLICATION

(11) **EP 3 348 289 A1**
(43) Date of publication of application: **18.07.2018**
(21) Application number: 18150022.4
(22) Date of filing: 02.01.2018
(51) Int. Cl.: A61M 1/00

(54) **SUCTION DISC**

(30) Priority: 13.01.2017 CN 201710024927
(71) Applicant: Suneetek(Xiamen) Medical Equipment Co., Ltd., 361000 Xiamen City, Fujian China (CN)
(72) Inventor: CHEN, Chi-Yuan, 205 Keelung City, Taiwan, (TW)
(74) Representative: Winter, Brandl, Fürniss, Hübner, Röss, Kaiser, Polte - Partnerschaft mbB

(57) **Abstract**

A suction disc is configured for abutting against a dressing covering over a wound. The suction disc includes a base plate and a case. The base plate has a first surface and a second surface opposite to each other. The first surface abuts against the dressing. The base plate has a first opening and a second opening. The first opening and the second opening respectively communicates with the first surface. The case has a first chamber and a second chamber. The case communicates with the second surface. The first chamber communicates with the first opening. The second chamber communicates with the second opening. The suction disc has a channel disposed inside a sidewall of the case surrounding the first chamber and penetrating through the base plate. An end of the channel communicates with the first chamber. Another end of the channel communicates with the first surface.

## Description

### BACKGROUND

### Technical Field

The present disclosure relates to suction discs. More particularly, the present disclose relates to suction discs utilized for negative pressure wound therapy (NPWT).

### Description of Related Art

The negative pressure wound therapy (NPWT) is an adjuvant treatment for accelerating the healing of wounds, which has been becoming increasingly popular in recent years. The feature of this adjuvant treatment is to apply a negative pressure on the wound, such that the surface of the wound becomes smaller under an even distribution of the negative pressure. In addition, the body fluid can be guided and any local defect of the wound can be filled up. Moreover, excessive exudate can be removed. Furthermore, a wet but not too wet environment is provided for the closure of the wound, such that the edema of the tissue around the wound is alleviated and the growth of the granulation tissue is stimulated.

At present, negative pressure wound therapy can be broadly applied to many acute and chronic wounds in clinical treatment, including orthopedics, soft tissues, skin transplantation, pressure sores, venous leg ulcer, diabetic foot, surgical infection and postoperative wound etc. A doctor will access factors like the size, the depth, the exudate of the wound, whether the wound is infected etc., and combine with the past history of disease, the current physiological condition, the medication status and the autonomy etc., to comprehensively consider and to tell the patient if he is appropriate to receive the treatment of negative pressure wound therapy.

### SUMMARY

A technical aspect of the present disclosure is to provide a suction disc, which can allow the user to grasp more readily the effect of the negative pressure in the second chamber.

According to an embodiment of the present disclosure, a suction disc configured for abutting against a dressing covering over a wound is provided. The suction disc includes a base plate and a case. The base plate has a first surface and a second surface opposite to each other. The first surface is configured for abutting against the dressing. The base plate further has at least one first opening and at least one second opening. The first opening and the second opening respectively communicate with the first surface. The case has at least one first chamber and at least one second chamber. The case communicates with the second surface. The first chamber communicates with the first opening. The second chamber communicates with the second opening. The suction disc has at least one channel. The channel is at least partially disposed inside a sidewall of the case surrounding the first chamber and the channel at least partially penetrates through the base plate. An end of the channel communicates with the first chamber. Another end of the channel communicates with the first surface.

In one or more embodiments of the present disclosure, the base plate further has at least one first groove. The first groove is located on the first surface. An end of the first groove communicates with the channel. Another end of the first groove at least partially extends towards an edge of the base plate.

In one or more embodiments of the present disclosure, the base plate has at least one second groove. The second groove is located on the first surface. The second groove at least partially extends around the first opening and intersects with the first groove.

In one or more embodiments of the present disclosure, a quantity of the second groove is plural. The base plate further has at least one third groove. The third groove is located on the first surface. The third groove communicates with the adjacent second grooves.

In one or more embodiments of the present disclosure, the base plate further has at least one supporting rib. The supporting rib protrudes from the first surface. The supporting rib extends on the first surface and is at least partially adjacent to the second groove.

In one or more embodiments of the present disclosure, the base plate further has at least one supporting rib. The supporting rib protrudes from the first surface. The supporting rib extends on the first surface.

In one or more embodiments of the present disclosure, the supporting rib at least partially surrounds the second opening.

In one or more embodiments of the present disclosure, the channel, the end of the channel communicating with the first chamber, the other end of the channel communicating with the first surface align as a straight line.

In one or more embodiments of the present disclosure, the case further has a first connecting hole and a second connecting hole. The first connecting hole and the second connecting hole respectively communicate with the first chamber and the second chamber. The suction disc further comprises a first connecting duct and a second connecting duct. The first connecting duct connects with the first connecting hole and communicates with the first chamber. The second connecting duct connects with the second connecting hole and communicates with the second chamber.

In one or more embodiments of the present disclosure, the base plate further has at least one positioning column. The positioning column protrudes from the first surface.

When compared with the prior art, the above-mentioned embodiments of the present disclosure have at least the following advantages:
(1) The channel is at least partially disposed inside a sidewall of the case surrounding the first chamber, and the channel at least partially penetrates through the base plate. In addition, an end of the channel communicates with the first chamber. Another end of the channel communicates with the first surface. In other words, even if the first opening communicated with the first chamber is blocked because the volume of the body fluid absorbed by the dressing is excessive or the body fluid becomes more viscous, the first chamber still communicates through the channel with the dressing and thus the second chamber. In this way, with the communication between the first chamber and the first surface by the channel, the chance that the first chamber communicates with the second chamber through the dressing will be effectively increased. This facilitates the user to grasp the condition of the absorption of the body fluid by the dressing. Thus, the user can grasp more readily the effect of the negative pressure in the second chamber.
(2) The first grooves are located on the first surface. An end of each of the first grooves communicates with the channel. Another end of each of the first grooves at least partially extends towards an edge of the base plate. Structurally speaking, the first grooves increase the chance of mutual communication between the channel and the dressing. Therefore, the chance that the first chamber communicates with the second chamber through the dressing will be effectively increased. This facilitates the user to grasp the condition of the absorption of the body fluid by the dressing. Thus, the user can grasp more readily the effect of the negative pressure in the second chamber.

### BRIEF DESCRIPTION OF THE DRAWINGS

The disclosure can be more fully understood by reading the following detailed description of the embodiments, with reference made to the accompanying drawings as follows:
Fig. 1 is a schematic view of application of a suction disc according to an embodiment of the present disclosure;
Fig. 2 is a cross-sectional view along the section line X of Fig. 1; and
Fig. 3 is a bottom view of the suction disc of Fig. 1.

### DETAILED DESCRIPTION

Drawings will be used below to disclose embodiments of the present disclosure. For the sake of clear illustration, many practical details will be explained together in the description below. However, it is appreciated that the practical details should not be used to limit the claimed scope. In other words, in some embodiments of the present disclosure, the practical details are not essential. Moreover, for the sake of drawing simplification, some customary structures and elements in the drawings will be schematically shown in a simplified way. Wherever possible, the same reference numbers are used in the drawings and the description to refer to the same or like parts.

Unless otherwise defined, all terms (including technical and scientific terms) used herein have the same meanings as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

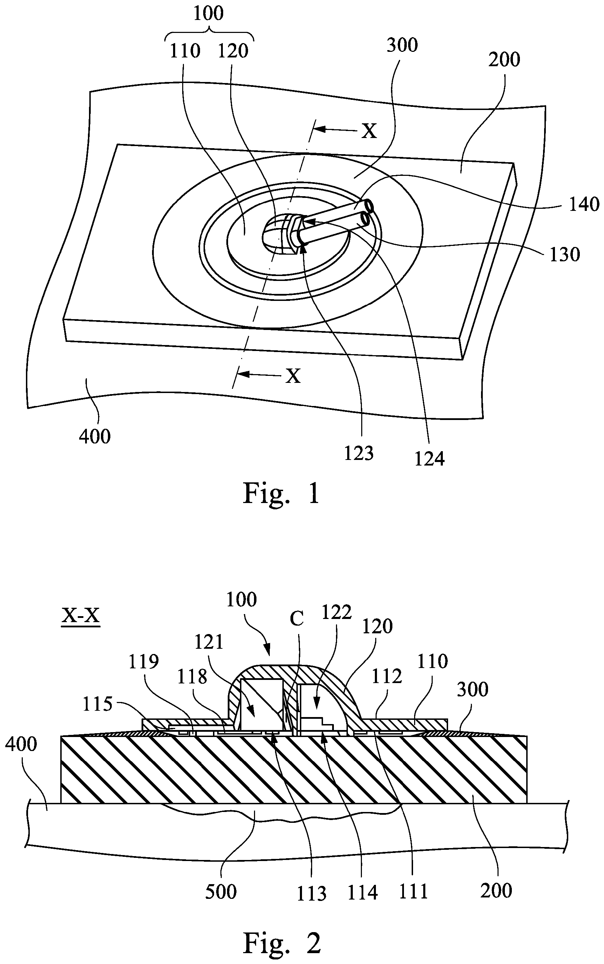

Reference is made to Fig. 1. Fig. 1 is a schematic view of application of a suction disc 100 according to an embodiment of the present disclosure. As shown in Fig. 1, a suction disc 100 is configured for being utilized in a negative pressure wound therapy (NPWT) to abutting against a dressing 200 covering over a wound 500 (please refer to Fig. 2). In practical applications, the dressing 200 is covered over the wound 500 of the patient 400. Afterwards, the suction disc 100 is disposed on a position of the dressing 200 corresponding to the wound 500. This means the dressing 200 is located between the suction disc 100 and the wound 500 of the patient 400. Furthermore, with a sticking film 300 adhered between the dressing 200 and the suction disc 100 around the wound 500, the relative position between the suction disc 100 and the dressing 200 is effectively fixed. In addition, the wound 500 is protected by the suction disc 100 and the sticking film 300, such that the wound 500 is not exposed to the air, thus reducing the chance of infection of the wound 500. In this embodiment, the suction disc 100 includes a base plate 110 and a case 120.

Please refer to Fig. 2. Fig. 2 is a cross-sectional view along the section line X of Fig. 1. To be more specific, as shown in Fig. 2, the base plate 110 has a first surface 111 and a second surface 112 opposite to each other. The first surface 111 is configured for abutting against the dressing 200. The base plate 110 further has at least one first opening 113 and at least one second opening 114. The first opening 113 and the second opening 114 respectively communicate with the first surface 111. The case 120 has at least one first chamber 121 and at least one second chamber 122. The case 120 communicates with the second surface 112. The first chamber 121 communicates with the first opening 113. The second chamber 122 communicates with the second opening 114. The suction disc 100 has at least one channel C. The channel C is at least partially disposed inside a sidewall of the case 120 surrounding the first chamber 121, and the channel C at least partially penetrates through the base plate 110. In addition, an end of the channel C communicates with the first chamber 121. Another end of the channel C communicates with the first surface 111.

In practical applications, the second chamber 122 communicates with an external negative pressure device (not shown). The negative pressure device is configured for providing a negative pressure to the second chamber 122. In the process of treatment, exudation from the wound 500 occurs continuously. The regional negative pressure generated by the suction disc 100 in the second chamber 122 is able to suck the infected substances and the excessive tissue exudate from the wound 500, so as to maintain the wettability of the wound 500, reduce the range of edge of the wound 500, and increase the flow volume of blood of the capillaries in the vicinity. Furthermore, the cells and thus the angiogenesis are stimulated by the mechanical stress generated by the dressing 200. Thus, the growth of the granulation tissue is promoted. All these mechanisms work together to speed up the healing process of the wound 500.

In addition, the first chamber 121 communicates with an external sensing device (not shown). The sensing device is configured for sensing the pressure inside the first chamber 121. To be more specific, the dressing 200 can be a porous material. This means that the first chamber 121 and the second chamber 122 can communicate with each other through the dressing 200. In other words, when the negative pressure device provides a negative pressure to the second chamber 122, the air in the first chamber 121 will flow to the second chamber 122 through the dressing 200. However, after the exudate from the wound 500 is gradually absorbed into the dressing 200, the process that the air in the first chamber 121 flows to the second chamber 122 through the dressing 200 will gradually become more difficult. As a result, the pressure inside the first chamber 121 will also be varied. With the variation of the pressure inside the first chamber 121 being sensed by the sensing device, the user can grasp the condition of the absorption of the body fluid by the dressing 200. Consequently, according to the actual situation, the user can adjust the magnitude of the negative pressure provided by the negative pressure device to the second chamber 122, or even replace with a new piece of dressing 200.

As mentioned above, the channel C is at least partially disposed inside a sidewall of the case 120 surrounding the first chamber 121, and the channel C at least partially penetrates through the base plate 110. In addition, an end of the channel C communicates with the first chamber 121. Another end of the channel C communicates with the first surface 111. In other words, even if the first opening 113 communicated with the first chamber 121 is blocked because the volume of the body fluid absorbed by the dressing 200 is excessive or the body fluid becomes more viscous, the first chamber 121 still communicates through the channel C with the dressing 200 and thus the second chamber 122. In this way, with the communication between the first chamber 121 and the first surface 111 by the channel C, the chance that the first chamber 121 communicates with the second chamber 122 through the dressing 200 will be effectively increased. This facilitates the user to grasp the condition of the absorption of the body fluid by the dressing 200. Thus, the user can grasp more readily the effect of the negative pressure in the second chamber 122.

In order to smoothen the flow of air in the channel C between the first chamber 121 and the dressing 200, in this embodiment, as shown in Fig. 2, the channel C, the end of the channel C communicating with the first chamber 121, the other end of the channel C communicating with the first surface 111 align as a straight line.

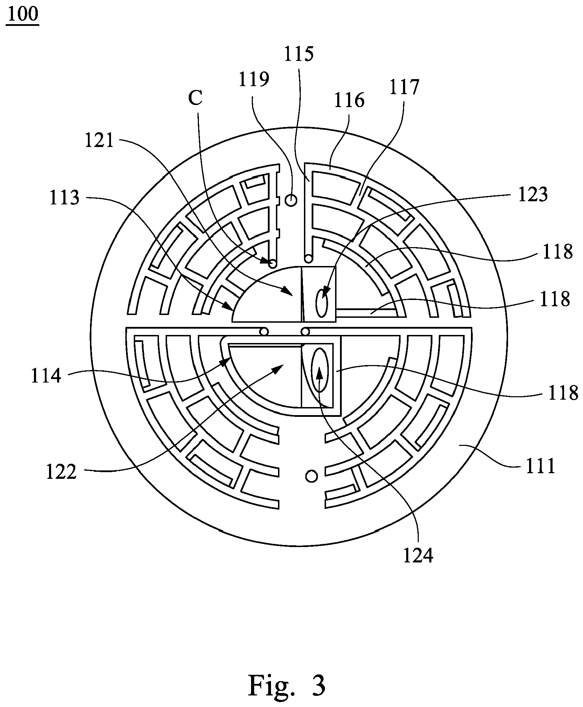

Please refer to Fig. 3. Fig. 3 is a bottom view of the suction disc 100 of Fig. 1. As shown in Fig. 3, the base plate 110 further has a plurality of first grooves 115. The first grooves 115 are located on the first surface 111. An end of each of the first grooves 115 communicates with the channel C. Another end of each of the first grooves 115 at least partially extends towards an edge of the base plate 110. Structurally speaking, the first grooves 115 increase the chance of mutual communication between the channel C and the dressing 200. Therefore, similarly, the chance that the first chamber 121 communicates with the second chamber 122 through the dressing 200 will be effectively increased. This facilitates the user to grasp the condition of the absorption of the body fluid by the dressing 200. Thus, the user can grasp more readily the effect of the negative pressure in the second chamber 122.

Furthermore, as shown in Fig. 3, the base plate 100 further has a plurality of second grooves 116. The second grooves 116 are located on the first surface 111. The second grooves 116 at least partially extend around the first opening 113 and intersect with the first grooves 115. Moreover, in this embodiment, the second grooves 116 are arranged on the first surface 111 in a direction away from the first opening 113. Structurally speaking, the second grooves 116 increase the chance of mutual communication between the channel C and the dressing 200. Therefore, similarly, the chance that the first chamber 121 communicates with the second chamber 122 through the dressing 200 will be effectively increased. This facilitates the user to grasp the condition of the absorption of the body fluid by the dressing 200. Thus, the user can grasp more readily the effect of the negative pressure in the second chamber 122.

In this embodiment, as shown in Fig. 3, the base plate 110 further has a plurality of third grooves 117. The third grooves 117 are located on the first surface 111. The third grooves 117 communicate between the adjacent second grooves 116 arranged along the direction away from the first opening 113. Structurally speaking, the third grooves 117 increase the chance of mutual communication between the channel C and the dressing 200. Therefore, similarly, the chance that the first chamber 121 communicates with the second chamber 122 through the dressing 200 will be effectively increased. This facilitates the user to grasp the condition of the absorption of the body fluid by the dressing 200. Thus, the user can grasp more readily the effect of the negative pressure in the second chamber 122.

As shown in Figs. 2-3, the base plate 110 further has at least one supporting rib 118. The supporting rib 118 protrudes from the first surface 111. The supporting rib 118 extends on the first surface 111. For example, in this embodiment, the supporting rib 118 is at least partially adjacent to the second grooves 116. Thus, a gap is formed between the second grooves 116 and the dressing 200, and the chance that the second grooves 116 are blocked by the dressing 200 is reduced.

On the other hand, the supporting rib 118 can also at least partially surround the second opening 114. Thus, a gap is formed between the second opening 114 and the dressing 200, and the chance that the second opening 114 is blocked by the dressing 200 is reduced.

In practical applications, as shown in Figs. 2-3, the base plate 110 further has at least one positioning column 119. The positioning column 119 protrudes from the first surface 111. Consequently, when the suction disc 100 is disposed on the dressing 200, the relative position between the suction disc 100 and the dressing 200 can be fixed.

As shown in Figs. 1 and 3, the case 120 further has a first connecting hole 123 and a second connecting hole 124. The first connecting hole 123 and the second connecting hole 124 respectively communicate with the first chamber 121 and the second chamber 122. The sensing device communicates with the first chamber 121 through the first connecting hole 123, while the negative pressure device communicates with the second chamber 122 through the second connecting hole 124. To be more specific, as shown in Fig. 1, the suction disc 100 further comprises a first connecting duct 130 and a second connecting duct 140. The first connecting duct 130 connects with the first connecting hole 123 and communicates with the first chamber 121. Moreover, the first connecting duct 130 communicates with the sensing device. The second connecting duct 140 connects with the second connecting hole 124 and communicates with the second chamber 122. Moreover, the second connecting duct 140 communicates with the negative pressure device.

In conclusion, when compared with the prior art, the aforementioned embodiments of the present disclosure have at least the following advantages.
(1) The channel is at least partially disposed inside a sidewall of the case surrounding the first chamber, and the channel at least partially penetrates through the base plate. In addition, an end of the channel communicates with the first chamber. Another end of the channel communicates with the first surface. In other words, even if the first opening communicated with the first chamber is blocked because the volume of the body fluid absorbed by the dressing is excessive or the body fluid becomes more viscous, the first chamber still communicates through the channel with the dressing and thus the second chamber. In this way, with the communication between the first chamber and the first surface by the channel, the chance that the first chamber communicates with the second chamber through the dressing will be effectively increased. This facilitates the user to grasp the condition of the absorption of the body fluid by the dressing. Thus, the user can grasp more readily the effect of the negative pressure in the second chamber.
(2) The first grooves are located on the first surface. An end of each of the first grooves communicates with the channel. Another end of each of the first grooves at least partially extends towards an edge of the base plate. Structurally speaking, the first grooves increase the chance of mutual communication between the channel and the dressing. Therefore, the chance that the first chamber communicates with the second chamber through the dressing will be effectively increased. This facilitates the user to grasp the condition of the absorption of the body fluid by the dressing. Thus, the user can grasp more readily the effect of the negative pressure in the second chamber.

## Claims

1. A suction disc (100) configured for abutting against a dressing (200) covering over a wound (500), the suction disc (100), **characterized by** comprising:
a base plate (110) having a first surface (111) and a second surface (112) opposite to each other, the first surface (111) being configured for abutting against the dressing (200), the base plate (110) further having at least one first opening (113) and at least one second opening (114), the first opening (113) and the second opening (114) respectively communicating with the first surface (111); and
a case (120) having at least one first chamber (121) and at least one second chamber (122), the case (120) communicating with the second surface (112), the first chamber (121) communicating with the first opening (113), and the second chamber (122) communicating with the second opening (114);
wherein the suction disc (100) has at least one channel (C) at least partially disposed inside a sidewall of the case (120) surrounding the first chamber (121) and at least partially penetrating through the base plate (110), an end of the channel (C) communicates with the first chamber (121), another end of the channel (C) communicates with the first surface (111).

2. The suction disc (100) of claim 1, **characterized in that** the base plate (110) further has at least one first groove (115) located on the first surface (111), an end of the first groove (115) communicates with the channel (C), another end of the first groove (115) at least partially extends towards an edge of the base plate (110).

3. The suction disc (100) of claim 2, **characterized in that** the base plate (110) has at least one second groove (116) located on the first surface (111), the second groove (116) at least partially extends around the first opening (113) and intersects with the first groove (115).

4. The suction disc (100) of claim 3, **characterized in that** a quantity of the second groove (116) is plural, the base plate (110) further has at least one third groove (117) located on the first surface (111), the third groove (117) communicates with the adjacent second grooves (116).

5. The suction disc (100) of claim 3, **characterized in that** the base plate (110) further has at least one supporting rib (118) protruding from the first surface (111), the supporting rib (118) extends on the first surface (111) and is at least partially adjacent to the second groove (116).

6. The suction disc (100) of claim 1, **characterized in that** the base plate (110) further has at least one supporting rib (118) protruding from the first surface (111), the supporting rib (118) extends on the first surface (111).

7. The suction disc (100) of claim 6, **characterized in that** the supporting rib (118) at least partially surrounds the second opening (114).

8. The suction disc (100) of claim 1, **characterized in that** the channel (C), the end of the channel (C) communicating with the first chamber (121), the other end of the channel (C) communicating with the first surface (111) align as a straight line.

9. The suction disc (100) of claim 1, **characterized in that** the case (120) further has a first connecting hole (123) and a second connecting hole (124) respectively communicating with the first chamber (121) and the second chamber (122), the suction disc (100) further comprises a first connecting duct (130) and a second connecting duct (140), the first connecting duct (130) connects with the first connecting hole (123) and communicates with the first chamber (121), the second connecting duct (140) connects with the second connecting hole (124) and communicates with the second chamber (122).

10. The suction disc (100) of claim 1, **characterized in that** the base plate (110) further has at least one positioning column (119) protruding from the first surface (111).
